# NEUE EUROPÄISCHE PATENTSCHRIFT

(11) **EP 1 017 359 B2**
(45) Veröffentlichungstag und Bekanntmachung des Hinweises auf die Entscheidung über den Einspruch: **09.07.2008**
(45) Hinweis auf die Patenterteilung: 29.12.2004
(21) Anmeldenummer: 99940041.9
(22) Anmeldetag: 23.07.1999
(51) Int. Cl.: A61K 8/84, A61Q 5/06

(54) **HAARFESTIGENDE MITTEL MIT AMPHOTEREN UND SAUREN POLYMEREN**
HAIR-SETTING AGENT WITH AMPHOTERIC AND ACIDIC POLYMERS
AGENT FIXATIF CONTENANT DES POLYMERES AMPHOTERES ET ACIDES DESTINE AUX CHEVEUX

(30) Priorität: 25.07.1998 DE 19833516
(43) Veröffentlichungstag der Anmeldung: 12.07.2000
(73) Patentinhaber: Wella Aktiengesellschaft, 64274 Darmstadt (DE)
(72) Erfinder: KARLEN, Thomas, CH-3013 Bern (CH); JAHEDSHOAR, Mehrdad, Studiocity, CA 91604 (US)
(86) Internationale Anmeldenummer: PCT/EP1999/005289
(87) Internationale Veröffentlichungsnummer: WO 2000/006092

(56) Entgegenhaltungen:
- EP-A- 0 797 979
- EP-B- 0 283 817
- EP-B- 0 521 666
- WO-A-94/01076
- WO-A-94/01077
- WO-A-96/36312
- WO-A-98/19653
- WO-A-99/22698
- WO-A-99/34770
- DE-A- 3 044 754
- DE-A- 3 929 973
- FR-A- 2 750 047
- US-A- 4 814 101
- Ausdruck aus der CTFA On-Line Datenbank zum Stichwort "Carbomer"

## Beschreibung

Gegenstand der Erfindung ist ein haarfestigendes Mittel mit einem Gehalt an bestimmten amphoteren Polymeren und bestimmten Polymeren, welche Säuregruppen aufweisen.

Haarstylingprodukte dienen dazu, die Haare in Form zu bringen sowie das Haar zu pflegen. Säuregruppenhaltige Polymere sind in der Lage, der Frisur bereits in niedriger Konzentration einen festen Halt zu verleihen. Sie besitzen jedoch den Nachteil, daß sie dem nassen Haar keine gute Kämmbarkeit verleihen. Kationische Polymere oder Polymere mit kationischen Gruppen sind sehr effiziente Kämmbarkeitsverbesserer, jedoch liegt ihre Festigungskraft wesentlich unter derjenigen der säuregruppenhaltigen Polymere. Zudem neigen kationische Polymere dazu, sich auch über Haarwäschen hinweg am Haar anzureichern, was dazu führen kann, daß das Haar einen stumpfen und belasteten Aspekt erhält.

Um die Naßkämmbarkeit von Haaren zu verbessern, werden im allgemeinen Zusatzstoffe wie Öle oder kationische niedrigmolekulare Stoffe verwendet. Die Öle (insbesondere auch Silikonöle) besitzen jedoch den Nachteil, daß sie als Weichmacher die Festigung der Frisur herabsetzen und zusätzlich dem Haar eine störende Belastung geben.

Werden säuregruppenhaltige Polymere mit kationischen Zusatzstoffen versetzt, erfolgt normalerweise eine nicht erwünschte spontane Ausfällung des Polymers.

Die FR 2 750 047 A1 beschreibt kosmetische Zusammensetzungen in Form von hochviskosen wässrigen Gelen. Das Beispiel 2 ist ein Gel zur Haarbehandlung und enthält ein erstes Terpolymer aus Dimethyldiallylammoniumchlorid, Acrylsäure und Acrylamid sowie ein zweites Terpolymer aus Methacrylsäure, C8-22-Alkylacrylat und polyethoxyliertem C1-22-Alkyl-allylether. Die EP 0 797 979 A1 beschreibt kosmetische Zusammensetzungen welche ein haarfestigendes Polymer und eine amphotere Stärke enthalten. Beispiel 4 ist ein Gel zur Haarbehandlung und enthält ein Terpolymer aus Dimethyldiallylammoniumchlorid, Acrylsäure und Acrylamid sowie vernetzte Polyacrylsäure.

Es bestand somit die Aufgabe, ein Haarbehandlungsmittel zu finden, welches den behandelten Haaren einen festen Halt verleiht und gleichzeitig haarpflegende Eigenschaften, insbesondere eine gute Naßkämmbarkeit besitzt ohne das Haar zu belasten und welches die oben genannten Nachteile nach Möglichkeit nicht aufweist. Es wurde nun gefunden, daß die Mischung aus geeigneten säuregruppenhaltigen Polymeren mit ausgewählten amphoteren Polymeren sowohl dem Haar im nassen Zustand eine ausgezeichnete Naßkämmbarkeit verleiht als auch der Frisur nach dem Trocknen einen festen Halt gibt, ohne daß das Haar belastet wird.

Gegenstand der Erfindung ist ein haarfestigendes Mittel in Form eines Aerosol-oder Nonaerosol-Haarsprays, eines Haarschaumes, eines Haargeles, eines Haarwachses, einer Haarcreme oder einer Haarlotion mit einem Gehalt an
(A) mindestens einem amphoteren Polymer, welches ausgewählt ist aus Copolymeren von Acrylsäure, Methylacrylat und Methacrylamidopropyltrimethylammoniumchlorid, Copolymeren aus Acrylamidopropyltrimethylammoniumchlorid und Acrylaten, Copolymeren aus Acrylamid, Acrylamidopropyltrimethylammoniumchlorid, 2-Amidopropylacrylamidsulfonat und Dimethylaminopropylamin,
(B) mindestens einem Polymeren, welches keine quaternären Amingruppen enthält und gebildet ist aus mindestens einer Monomerart, welche Säuregruppen aufweist und wobei das Polymer ausgewählt ist aus vernetzten oder nicht vernetzten Vinylacetat/ Crotonsäure Copolymeren, Acrylsäure/Alkylacrylat/ N-Alkylacrylamid Copolymeren, partialveresterte Copolymere zwischen Vinylmethylether und Maleinsäureanhydrid und Vinylacetat/Crotonat/Vinylalkanoat Copolymeren,
wobei die Säuregruppen der Polymere (A) und (B) in neutralisierter oder in nicht neutralisierter Form vorliegen können;
ausgenommen einer Mousse-Zusammensetzung mit einem Gehalt an 6 Gew.% Ethanol, 4 Gew.% Butylester von Vinylmethylether/Maleinsäureanhydrid Copolymer (50%ig), 2 Gew.% PVP/VA (50%ig), 0,1 Gew.% Parfüm, 83,19 Gew.% Wasser, 0,1 Gew.% Aminomethylpropanol, 0,3 Gew.% Polyquaternium-47, 0,3 Gew.% Laurinsäureethanolamid, 0,1 Gew.% Steareth-21, 3 Gew.% Silikonmikroemulsion (25%ig) und 0,91 Gew.% Konservierungsmittel.

Das amphotere Polymer (A) liegt vorzugsweise in einer Menge von 0,01 bis 5 Gewichtsprozent, besonders bevorzugt in einer Menge von 0,05 bis 3 Gewichtsprozent in dem erfindungsgemäßen Mittel vor und enthält vorzugsweise im wesentlichen keine Monomere mit Betainstruktur. Das Polymer (B) mit Säuregruppen liegt vorzugsweise in einer Menge von 0,01 bis 20 Gewichtsprozent, besonders bevorzugt in einer Menge von 0,1 bis 10 Gewichtsprozent in dem erfindungsgemäßen Mittel vor.

Die Säuregruppen der Polymere (A) und(B) können in dem erfindungsgemäßen Haarbehandlungsmittel unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH und andere.

Die Polymere (A) und (B) werden so gewählt, daß sie miteinander verträglich sind. Unter dem Begriff 'verträglich' wird verstanden, daß bei Mischung von Polymer (A) mit Polymer (B) keine spontane Ausklumpung erfolgen soll. Die Polymermischung soll im Haarbehandlungsmittel so vorliegen, daß das erfindüngsgemäße Mittel durch handelsübliche Applikationsformen leicht auf dem Haar verteilbar ist und einen unsichtbaren, festigenden Film auf dem Haar ausbildet, ohne daß auf dem Haar eine Verklumpung festzustellen ist.

Das erfindungsgemäße Mittel wird bevorzugt in einem wäßrigen oder in einem wäßrig-alkoholischen Milieu mit mindestens 10 Gewichtsprozent Wasser konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Desweiteren können Lösungsmittel oder ein Gemisch aus Lösungsmitteln mit einem Siedepunkt unter 400°C in einer Menge von 0,1 bis 90 Gewichtsprozent bevorzugt von 1 bis 50 Gewichtsprozent eingesetzt werden. Besonders geeignet sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin und Propylenglykol in einer Menge bis 30 Gewichtsprozent.

Das erfindungsgemäße Mittel kann in einem pH-Bereich von 2,0 bis 9,5 vorliegen. Besonders bevorzugt ist der pH-Bereich zwischen 2,5 und 8. Liegt das erfindungsgemäße Mittel im sauren Bereich vor, so kann es organische oder anorganische Säuren enthalten wie beispielsweise Ameisensäure, Weinsäure, Äpfelsäure, Maleinsäure, Fumarsäure, Ameisensäure, Pyrrolidoncarbonsäure, Zitronensäure, Schwefelsäure, Essigsäure, Salzsäure, Phosphorsäure u.a..

Das amphotere Polymer (A) ist ein Copolymer, welches gebildet ist aus mindestens einer ersten Monomerart, welche quaternäre Amingruppen aufweist und mindestens einer zweiten Monomerart, welche Säuregruppen aufweist.

Geeignete Monomere eines amphoteren Copolymers (A), welche quaternäre Amingruppen aufweisen, sind Acrylamidopropyltrimethylammoniumchlorid und Methacrylamidopropyltrimethylammoniumchlorid.

Geeignete Monomere des amphoteren Copolymers (A), welche Säuregruppen aufweisen, sind ungesättigte, radikalisch polymerisierbare Verbindungen, welche mindestens eine Säuregruppe tragen, insbesondere Carboxyvinylmonomere wie z.B. Acrylsäure und Methacrylsäure.

Beispiele für als Komponente (A) geeignete Copolymere sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat® 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung Bozequat® 4000 vertrieben werden.

Polymere (B) mit Säuregruppen sind vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET® CA-66 vertrieben werden. Ebenso bevorzugt sind partialveresterte Copolymere zwischen Vinylmethylether und Maleinsäureanhydrid, wie sie unter der Handelsbezeichnung Gantrez® von der Firma ISP vertrieben werden. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z. B. von der Firma National Starch unter der Handelsbezeichnung RESYN 28-2930 vertrieben werden.

Eine bevorzugte Anwendungsform des erfindungsgemäßen Mittels enthält zusätzlich kationische Kämmbarkeitsverbesserer, insbesondere kationische Tenside, kationische Polymere oder kationische Silikonverbindungen. Die Kämmbarkeitsverbesserer sind in einer Menge von vorzugsweise 0.05 bis 10 Gewichtsprozent, besonders bevorzugt von 0,1 bis 3 Gewichtsprozent enthalten.

Geeignete kationische Tenside besitzen die allgemeine Formel [NR¹R²R³R⁴]⁺ X⁻, wobei die Reste R¹, R², R³ und R⁴ unabhängig voneinander eine aliphatische Gruppe mit 1 bis 22 Kohlentoffatomen oder eine aromatische, Alkoxy-, Polyoxyalkylen-, Alkylamido-, Hydroxyalkyl-, Aryl-oder Alkylarylgruppe mit bis zu 22 Kohlenstoffatomen bedeutet und X ein salzbildendes Anion ist, beispielsweise Halogenide wie Chlorid oder Bromid, oder Acetat, Citrat, Lactat, Glykolat, Phosphat, Nitrat, Sulfat oder Alkylsulfat. Die langkettigen aliphatischen Gruppen, d.h. solche mit rund 12 oder mehr Kohlenstoffatomen können gesättigt oder ungesättigt sein. Bevorzugt sind kationische Tenside mit entweder einer langkettigen und drei kurzkettigen oder mit zwei langkettigen und zwei kurzkettigen aliphatischen Gruppen, wobei die langkettigen Gruppen 12 bis 22, vorzugsweise 16 bis 22 Kohlenstoffatome und die kurzkettigen Gruppen 1 bis 3, vorzugsweise 1 bis 2 Kohlenstoffatome aufweisen. Bevorzugte kationische Tenside sind Cetyltrimethylammoniumchlorid, Behenyltrimethylammoniumchlorid, Stearyltrimethylammoniumchlorid, Stearyldimethylbenzylammoniumchlorid, Cetylpyridiniumchlorid, Ditallowdimethylammoniumchlorid, Monotallowtrimethylammoniumchlorid und Dicetyldimethylammoniumchlorid oder deren Gemische.

Geeignete kationische Silikonverbindungen sind insbesondere mit quaternären Aminogruppen endfunktionalisierte Silikonpolymere, beispielsweise die unter den Handelsbezeichnungen Abil® Quat 3270, Abil® Quat 3272 und Abil® Quat 3474 von der Firma, Goldschmidt vertriebenen Produkte mit der INCI Bezeichnung Quaternium-80.

Bevorzugte Anwendungsformen des erfindungsgemäßen Mittels enthalten als weitere Komponente 0,01 bis 50 Gewichtsprozent, vorzugsweise 0,5 bis 50 Gewichtsprozent mindestens eines synthetischen oder natürlichen, filmbildenden, nichtionischen oder kationischen haarfestigenden Polymers. Die haarfestigenden Polymere können einzeln oder in einem Gemisch eingesetzt werden. Desweiteren können Polymere mit verdickender Wirkung eingesetzt werden.

Unter filmbildenden, haarfestigenden Polymeren sollen solche Polymere verstanden werden, welche allein in 0,1 bis 5%iger wäßriger, alkoholischer oder wäßrig-alkoholischer Lösung angewandt, in der Lage sind, auf dem Haar einen Polymerfilm abzuscheiden und auf diese Weise das Haar zu festigen.

Geeignete synthetische, nichtionische, filmbildende, haarfestigende Polymere sind zum Beispiel Homopolymere des Vinylpyrrolidons, die beispielsweise unter den Handelsbezeichnungen LUVISKOL® K von der Firma BASF, Deutschland oder PVP-K von der Firma ISP, USA vertrieben werden, sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind zum Beispiel Copolymerisate aus Vinylpyrrolidon und Vinylacetat, die beispielsweise unter den Handelsbezeichnungen LUVISKOL® VA von der Firma BASF/ Deutschland vertrieben werden; Terpolymere aus Vinylpyrrolidon, Vinylacetat und Vinylpropionat, die beispielsweise unter der Handelsbezeichnung LUVISKOL® VAP der Firma BASF/Deutschland vertrieben werden; Polyacrylamide, die beispielsweise unter den Handelsbezeichnungen AKYPOMINE® P 191 von der Firma CHEM-Y/ Deutschland oder SEPIGEL® 305 von der Firma SEPPIC/USA vertrieben werden; Polyvinylalkohole, die beispielsweise unter den Handelsbezeichnungen ELVANOL® der Firma Du Pont oder VINOL® 523/540 der Firma Air Products/USA vertrieben werden, sowie hochmolekulares Polyethylenglykol oder hochmolekulare Copolymere von Ethylenglykol mit Propylenglykol mit festigenden Eigenschaften, die beispielsweise unter den Handelsbezeichnungen LIPOXOL® 1000 von der Firma HÜLS AG/Deutschland, PLURACOL E 4000 von der Firma BASF/Deutschland oder UPIWAX® 20.000 von der Firma UPI vertrieben werden.

Geeignete natürliche filmbildende Polymere mit haarfestigender Wirkung sind zum Beispiel Chitosan mit einem Molekulargewicht von 20.000 bis ca. 5 Millionen g/mol, wie es beispielsweise von der Firma Pronova vertrieben wird oder verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel,Hydroxypropylcellulose mit einem Molekulargewicht von 30.000 bis 50.000 g/mol, welche beispielsweise unter der Handelsbezeichnung NISSO SL® von der Firma Lehmann & Voss/Deutschland vertrieben wird.

Von den kationischen Polymeren, die in dem erfindungsgemäßen Mittel enhalten sein können, ist zum Beispiel Polyvinylpyrrolidon/Dimethylaminoethylmethacrylat Copolymer, das unter der Handelsbezeichnung Gafquat® 755 N von der Firma Gaf Co./USA vertrieben wird, geeignet. Weitere kationische Polymere sind beispielsweise das von der Firma BASF AG/Deutschland unter dem Handelsnamen LUVIQUAT® HM 550 vertriebene Copolymer aus Polyvinylpyrolidon und Imidazoliminmethochlorid, das von der Firma Calgon/USA unter dem Handelsnamen Merquat® Plus 3300 vertriebene Terpolymer aus Dimethyldiallylammoniumchlörid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Amerchol/USA, unter dem Handelsnamen Polymer IR® vertriebene quaternierte Ammoniumsalz der Hydroxyethylcellulose und einem trimethylammonium-substituierten Epoxid und das von der Firma Gaf unter dem Handelsnamen Gafquat® HS 100 vertriebene Vinylpyrrolidon/Methacrylamidopropyltrimethylammoniumchlorid Copolymer.

Von den Verdickern, die in dem erfindungsgemäßen Mittel enthalten sein können, sind Homopolymere der Acrylsäure mit einem Molekulargewicht von 2.000.000 bis 6.000.000 zu nennen, die beispielsweise von der Firma BF Goodrich/USA unter der Handelsbezeichnung Carbopol® vertrieben werden. Als weitere Verdicker kann das erfindungsgemäße Mittel ein Acrylsäurehomopolymer mit einem Molekulargewicht von 4.000.000 enthalten, das beispielsweise von der Firma BF Goodrich unter der Handelsbezeichnung Carbopol® 940 vertrieben wird. Weitere Verdicker sind beispielsweise die von der Firma BF Goodrich unter dem Handelsnamen Carbopol® ETD 2001 oder von der Firma Protex/Frankreich unter dem Handelsnamen Modarez V 600 PX vertriebene Acrylsäurehomopolymer, das von der Firma Hoechst/ Deutschland unter dem Handelsnamen Hostacerin® PN 73 vertriebene Polymer aus Acrylsäure und Acrylamid (Natriumsalz) mit einem Molekulargwicht von 2.000.000 bis 6.000.000 und das von der Firma Alban Muller, Montreuil/Frankreich unter dem Handelsnamen Amigel® vertriebene Sclerotium Gum. Besonders bevorzugt sind die Copolymere der Acrylsäure oder der Methacrylsäure, wie sie zum Beispiel unter dem Handelsnamen Carbopol 1342 oder Pemulen TR1 der Firma GOODRICH, USA vertrieben werden.

Selbstverständlich können die erfindungsgemäßen Mittel auch weitere übliche kosmetische Zusätze, wie beispielsweise nichtfestigende nichtionische Polymere, wie zum Beispiel Polyethylenglykole oder Copolymere aus Ethylenglykol und Propylenglykol, nichtfestigende anionische Polymere und nichtfestigende natürliche Polymere sowie deren Kombination in einer Menge von vorzugsweise 0,01 - 50 Gewichtsprozent; Parfümöle in einer Menge von vorzugsweise 0,01 - 5 Gewichtsprozent; Trübungsmittel wie zum Beispiel Ethylenglykoldistearat, in einer Menge von vorzugsweise 0,01 - 5 Gewichtsprozent; Netzmittel oder Emulgatoren aus den Klassen der anionischen, kationischen, amphoteren oder nichtionogenen oberflächenaktiven Substanzen wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 - 30 Gewichtsprozent; ferner Feuchthaltemittel, Farbstoffe, Lichtschutzmittel, Antioxidantien, Glanzgeber und Konservierungsstoffe in einer Menge von vorzugsweise 0,01 - 10 Gewichtsprozent enthalten.

Als weitere Zusätze sind geeignete Silikonpolymere einsetzbar: Polydimethylsiloxan (INCI: Dimethicon), α-Hydro-ω-hydroxypolyoxydimethylsilylen (INCI: Dimethiconol), cyclisches Dimethylpolysiloxan (INCI: Cyclomethicon), Trimethyl(octadecyloxy)silan (INCI: Stearoxytrimethylsilan), Dimethylsiloxan-Glykol-Copolymer (INCI: Dimethicon Copolyol), Dimethylsiloxanaminoalkylsiloxan-Copolymer mit Hydroxyendgruppen (INCI: Amodimethicon), Monomethylpolysiloxan mit Laurylseitenketten und Polyoxyethylen- und/oder Polyoxypropylenendketten, (INCI: Laurylmethicon Copolyol), Dimethylsiloxan-Glykol-Copolymer-acetat (INCI: Dimethiconcopolyol Acetat) Dimetylsiloxanaminoalkylsiloxan-Copolymer mit Trimethylsilylendgruppen (INCI: Trimethylsilylamodimethicon). Bevorzugte Silikonpolymere sind: Dimethicone, welche beispielsweise von der Firma Wacker, München/Deutschland unter der Handelsbezeichnung SILOXANE F-221 oder von der Firma Dow Corning Europe, Brüssel/Belgien unter der Handelsbezeichnung Dow Corning Fluid 200/0,65 cs vertrieben werden; Cyclomethicone, die beispielsweise unter den Handelsbezeichnungen DOW CORNING 244 Fluid von der Firma Dow Corning Europe, Brüssel/Belgien oder ABIL® K4 von der Firma Goldschmidt/Deutschland vertrieben werden; Dimethiconole, die beispielsweise unter den Handelsbezeichnungen SILICONE FLUID F-212 von der Firma Wacker/Deutschland oder UNISIL® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet wie zum Beispiel eine Mischung aus Dimethicon und Dimethiconol, die beispielsweise unter der Handelsbezeichnung DOW CORNING 1403 Fluid von der Firma Dow Corning Europe/Belgien vertrieben wird.

Das erfindungsgemäße Mittel kann in verschiedenen Applikationsformen Anwendung finden, wie beispielsweise in Aerosolzubereitungen als Schaum oder als Spray, des weiteren als Non-Aerosol, welches mittels einer Pumpe oder als Pump-and-Spray zum Einsatz kommt. Der Einsatz in üblichen O/W und W/O Emulsionen ist ebenso möglich wie in Anwendungsformen als Gel, Wachs, Lotion oder Mikroemulsion. Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt. Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Wenn das erfindungsgemäße Mittel in Form eines versprühbaren Non-Aerosol-Haarsprays oder eines Non-Aerosol-Haarlacks vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeitohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel. infolge der Kontraktion des elastischen Behälters bei Öffnen des Sprühventils kontinuierlich abgegeben wird, verwendet werden.

Wenn das erfindungsgemäße Mittel in Form eines festigenden Haarschaumes (Mousse) vorliegt, so ist mindestens eines der Polymere schaumbildend oder es enthält zusätzlich mindestens eine für diesen Zweck bekannte, übliche schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Das erfindungsgemäße Mitttel weist als zusätzliche Komponente eine mechanische Vorrichtung zum Verschäumen der Zusammensetzung auf. Unter mechanischen Schäumvorrichtungen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Mittel in Form eines festigenden Haargels vorliegt, so enthält es zusätzlich mindestens eine gelbildende Substanz, beispielsweise die oben aufgeführten Verdickungsmittel in einer Menge von vorzugsweise 0,05 bis 10, besonders bevorzugt von 0,1 bis 2 Gewichtsprozent. Die Viskosität des Gels beträgt vorzugsweise von 500 bis 50.000 cSt, besonders bevorzugt von 1.000 bis 15.000 cSt bei 25°C.

Wenn das erfindungsgemäße Mittel in Form eines Haarwachses vorliegt, so enthält es zusätzlich wasserunlösliche Fettstoffe oder fett- bzw. wachsähnliche Stoffe in einer Menge von vorzugsweise 0,5 bis 30 Gewichtsprozent. Geeignete wasserunlösliche Stoffe sind beispielsweise Emulgatoren mit einem HLB-Wert unterhalb von 7, Silikonöle, Silikonwachse, Wachse (z.B. Wachsalkohole. Wachssäuren, Wachsester, sowie insbesondere natürliche Wachse wie Bienenwachs, Carnaubawachs, etc.), Fettalkohole, Fettsäuren oder Fettsäureester.

Wenn das erfindungsgemäße Mittel in Form einer festigenden Haarlotion vorliegt, so liegt es als nichtviskose Lösung, Dispersion oder Emulsion mit einem Gehalt an mindestens 10 Gewichtsprozent, vorzugsweise 20 bis 95 Gewichtsprozent eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden.

Wenn das erfindungsgemäße Mittel in Form einer festigenden Haarcreme, beispielsweise einer Färbecreme vorliegt, so liegt es vorzugsweise als Emulsion vor und enthält entweder zusätzlich viskositätsgebende Inhaltsstoffe wie die oben genannten Verdicker in einer Menge von 0,1 bis 10 Gewichtsprozent oder die erforderliche Viskosität und Konsistenz wird durch Micellbildung mit Hilfe von geeigneten Emulgatoren, Fettsäuren, Fettalkoholen, Wachsen etc. in üblicher Weise aufgebaut.

Desweiteren ist mit der erfindungsgemäßen Polymerkombination die Herstellung von Konzentraten möglich, welche einen verringerten Wasser- oder Lösungsmittelgehalt aufweisen. Die Konzentrate werden nach Transport und gegebenenfalls Lagerung durch Zugabe der erforderlichen Menge Wasser oder Lösungsmittel in ein anwendungsfähiges Haarbehandlungsmittel überführt.

Unter Haarbehandlung soll die Behandlung des menschlichen Kopfhaares vor allem zum Zweck der Herstellung einer Frisur oder zur Pflege der Haare verstanden werden.

Das erfindungsgemäße Mittel wird angewendet, indem eine für die gewünschte Festigung ausreichende Menge in oder auf dem trockenen Haar oder nach der Haarwäsche in oder auf dem feuchten, handtuchgetrochneten Haar verteilt wird. Die anzuwendende Menge hängt von der Haarfülle ab und beträgt typischerweise 5 bis 30 g. Das Mittel wird vorzugsweise nicht ausgespült, verbleibt also auf dem Haar. Anschließend wird das Haar gegebenenfalls durchgekämmt oder zur Frisur geformt und gegebenenfalls getrocknet. Die Frisurenerstellung kann aber auch bereits vor der Aufbringung des Mittels erfolgen, insbesondere wenn das Mittel in Form eines Sprays vorliegt.

Die nachfolgenden Beispiele sollen den Gegenstand der Erfindung näher erläutern.

### Beispiele

In den Beispielen wurden die folgenden Rohstoffe eingesetzt:

| Rohstoff | INCI-Bezeichnung |
|---|---|
| Gantrez® ES 225 | Ethyl Ester of PVM/MA Copolymer |
| Gantrez® ES 425 | Butyl Ester of PVM/MA Copolymer |
| Merquat® 2001 | Polyquaternium-47 |
| Aristoflex® A | VA/Crotonates Copolymer |
| Luviskol® VA 37 E | PVPNA Copolymer |
| Abil® Quat 3272 | Quaternium-80 |
| Luviquat® FC 905 | Polyquaternium-16 |
| Gafquat® 755 N | Polyquaternium-11 |
| Luviskol® K 80 | PVP |
| Rewoteric® AM CAS | Cocamidopropyl Hydroxysultaine |
| Amphomer® | Octylacrylamide/Acrylates/Butyl-aminoethyl Methacrylate Copolymer |
| Luviset® CA 66 | VA/Crotonates Copolymer |
| Oramix® NS 10 | Decyl Glucoside |
| Plex® 3073L | Polyquaternium-45 |
| Diaformer® Z-SM | Methacryloyl Ethyl Betaine / Acrylates Copolymer |

### Beispiel 1: Aerosolspray

| | |
|---|---|
| Gantrez® ES 425 (50% in Ethanol) | 10,00 g |
| Ethanol | 40,00 g |
| Wasser | 29,00 g |
| Merquat® 2001 (20% in Wasser) | 1,00 g |
| Dimethylether | 20,00 g |
| | 100,00 g |

### Beispiel 2: Nonaerosolspray

| | |
|---|---|
| Aristoflex® A; 40% in Ethanol | 2,00 g |
| Luviskol® VA 37 E | 3,00 g |
| Ethanol. | 70,00 g |
| Wasser | 24,50 g |
| Merquat® 2001 | 0,50 g |
| | 100,00 g |

### Beispiel 3: Nonaerosolspray mit höherem Wassergehalt

| | |
|---|---|
| Aristoflex® A; 40% in Ethanol | 4,00 g |
| Ethanol | 40,00 g |
| Wasser | 55,00g g |
| Merquat® 2001 | 1,00 g |
| | 100,00 g |

### Beispiel 4: Nonaerosolspray mit kationischem Zusatzstoff

| | |
|---|---|
| Aristoflex® A; 40% in Ethanol | 4,00 g |
| Ethanol | 40,00 g |
| Wasser | 54,50 g |
| Merquat® 2001 | 1,00 g |
| Abil® Quat 3272 | 0,50 g |
| | 100,00 g |

### Beispiel 5: Nonaerosolspray

| | |
|---|---|
| Aristoflex® A; 40% in Ethanol | 4,00 g |
| Aminomethylpropanol | 0,20 g |
| Ethanol | 60,00 g |
| Wasser | 34,80 g |
| Merquat® 2001 | 1,00 g |
| | 100,00 g |

### Beispiel 6: Nonaerosolspray mit kationischem Zusatzstoff

| | |
|---|---|
| Aristoflex® A; 40% in Ethanol | 4,00 g |
| Aminomethylpropanol | 0,20 g |
| Ethanol | 60,50 g |
| Wasser | 33,80 g |
| Merquat® 2001 | 1,00 g |
| Cetrimoniumchlorid | 0,50 g |
| | 100,00 g |

### Beispiel 7: Nonaerosolspray

| | |
|---|---|
| Gantrez® ES 225 (50% in Ethanol) | 5,00 g |
| Ethanol | 60,00 g |
| Wasser | 34,00 g |
| Merquat® 2001 | 1,00 g |
| | 100,00 g |

### Beispiel 8: Nonaerosolspray mit kationischem Polymer

| | |
|---|---|
| Aristoflex® A; 40% in Ethanol | 4,00 g |
| Luviquat® FC 905 | 3,00 g |
| Ethanol | 60,00 g |
| Wasser | 32,00 g |
| Merquat® 2001 | 1,00 g |
| | 100,00 g |

### Beisplel 9: Schaumfestiger

| | |
|---|---|
| Aristoflex® A; 40% in Ethanol | 5,00 g |
| Luviskol® K 80 | 1,00 g |
| Ethanol | 15,00 g |
| Wasser | 66,00 g |
| Merquat® 2001 | 2,00 g |
| Rewoteric® AM CAS | 1,00 g |
| PropanButan 5,0 bar | 10,00 g |
| | 100,00 g |

### Beispiel 10: Schaumfestiger mit kationischem Zusatzstoff

| | |
|---|---|
| Aristoflex® A; 40% in Ethanol | 5,00 g |
| Luviskol® K80 | 1,00 g |
| Ethanol | 15,00 g |
| Wasser | 65,50 g |
| Merquat® 2001 | 2,00 g |
| Abil® Quat 3272 | 1,00 g |
| Rewoteric® AM CAS | 0,50 g |
| Propan/Butan 5,0 bar | 10,00 g |
| | 100,00 g |

### Beispiel 11: Schaumfestiger

| | |
|---|---|
| Gantrez® ES 225 (50% in Ethanol) | 4, 00 g |
| Luviskol® K80. | 1,00 g |
| Ethanol | 15,00 g |
| Wasser | 67,00 g |
| Merquat® 2001 | 2,00 g |
| Rewoteric® AM CAS | 1,00 g |
| Propan/Butan 5,0 bar | 10,00 g |
| | 100,00 g |

### Beispiel 12: Schaumfestiger mit kationischem Polymer

| | |
|---|---|
| Aristoflex® A; 40% in Ethanol | 4,00 g |
| Gafquat® 755 N | 2,00 g |
| Ethanol. | 14,00 g |
| Wasser | 67,50 g |
| Merquat® 2001 | 2,00 g |
| Rewoteric® AM CAS | 0,50 g |
| Propan/Butan 5,0 bar | 10,00 g |
| | 100,00 g |

### Beispiel 13: Vergleichsversuche

Es wurden 5 Haarbehandlungsmittel 13-A1 bis 13-E1 mit einem Gehalt an jeweils einer erfindungsgemäßen Polymerkombination verglichen mit analogen Haarbehandlungsmitteln 13-A2 bis 13-E2, welche jeweils eine nicht erfindungsgemäße Polymerkombination enthielten. Die genauen Zusammensetzungen sind in Tabelle 1 wiedergegeben.

**Tabelle 1: Untersuchte Zusammensetzungen**

| Nummer | 13-A1 | 13-A2 | 13-B1 | 13-B2 | 13-C1 | 13-C2 | 13-D1 | 13-D2 | 13-E1 | 13-E2 |
|---|---|---|---|---|---|---|---|---|---|---|
| Applikationsform | Schaum | Schaum | Gel | Gel | Schaum | Schaum | Nonaerosol Spray | Nonaerosol Spray | Schaum | Schaum |
| Merquat^{®} 2001 (20% in Wasser) | 2.50 | - | 2.00 | - | 2.50 | - | 1.50 | - | 3.50 | - |
| Diaformer^{®} Z-SM (30%ig) | - | 1.66 | - | 1.33 | - | - | | | - | - |
| Amphomer^{®} | - | - | - | - | - | 0.50 | - | 0.3 | - | - |
| Plex^{®} 3073L (25%ig) | - | - | - | - | - | - | - | - | - | 2.80 |
| Luviset^{®} CA 66 | 2.50 | 2.50 | 2.00 | 2.00 | 2.50 | 2.50 | 2.50 | 2.50 | 2.00 | 2.00 |
| Aminomethylpropanol | 0.27 | 0.27 | pH7 | pH7 | 0.27 | 0.27 | Ad pH7 | Ad pH7 | 0.27 | 0.27 |
| Carbopol^{®} 980 | - | - | 1.00 | 1.00 | | | | | | |
| Oramix^{®} NS 10 | 0.10 | 0.10 | - | - | 0.20 | 0.20 | 0.10 | 0.10 | 0.10 | 0.10 |
| Ethanol | - | - | - | - | - | - | 30.00 | 30.00 | | |
| Wasser | Ad 100 | Ad 100 | Ad100 | Ad100 | Ad 100 | Ad 100 | Ad 100 | Ad 100 | Ad 100 | Ad 100 |
| Propan/Butan 4.8 | 8.00 | 8.00 | - | - | 8.00 | 8.00 | - | - | 8.00 | 8.00 |

In Halbseitenversuchen wurde jeweils eine Hälfte der Haare einer Testperson mit einem erfindungsgemäßen Haarbehandlungsmittel und die andere Hälfte der Haare mit einem nicht erfindungsmäßen Mittel behandelt. Die Versuche wurden für jeden Vergleich insgesamt sechsmal durchgeführt. Die behandelten Haare wurden durch ausgebildete Friseurfachkräfte sensorisch beurteilt. Dabei wurden die folgenden Beurteilungen erhalten:
Vergleich 13-A1 gegen 13-A2:
   Die Kämmbarkeit der Haare und der Haargriff sind bei Muster 13A1 deutlich besser. Die Festigung ist gleichwertig.
Vergleich 13-B1 gegen 13-B2:
   In trockenem und feuchtem Haar ist der Haargriff bei Muster 13-B1 besser. Die Nasskämmbarkeit ist für Muster 13-B1 besser, die Festigung ist gleichwertig.
Vergleich 13-C1 gegen 13-C2:
   Für Muster 13-C1 sind sowohl Kämmbarkeit als auch Haargriff und Festigung deutlich besser.
Vergleich 13-D1 gegen 13-D2:
   In feuchtem Haar sind Kämmbarkeit und Griff für Muster 13-D1 besser. Die Festigung ist gleichwertig.
Vergleich 13-E1 gegen 13-E2:
   Das amphotere, betainische Polymer Plex 3073L (Polyquaternium-45) ist in Muster 13-E2 nicht mit dem anionischen Polymer Luviset CA 66 (VA/Crotonates Copolymer) verträglich. Für Muster 13-E1 wurden keinerlei Verträglichkeitsprobleme festgestellt.

### Beispiel 14: Vergleichsversuche mit konstantem Polymergehalt (3,6 Gewichtsprozent)

In der folgenden Testreihe wurden haarfestigende Mittel mit einem Gehalt an einem der nachfolgend genannten Polymere sowie haarfestigende Mittel mit einem Gehalt an einer Zweierkombination der genannten Polymere untersucht, wobei die Polymere ausgewählt waren aus
- einem säuregruppenhaltigen Polymer (Abkürzung: Sa)
- einem amphoteren Polymer (Abkürzung: Am)
- einem nichtionischen Polymer (Abkürzung: Ni) sowie
- zwei kationischen Polymeren (Abkürzung: K1 und K2)

Es ergab sich folgende Kombinations-Matrix mit den Bezeichnungen A bis N für die nachfolgenden Rezepturen:

| | **Sa** | **Am** | **K1** | **K2** | **NI** |
|---|---|---|---|---|---|
| **Sa** | B | A | H | G | K |
| **Am** | A | C | M | N | L |
| **K1** | H | M | E | - | J |
| **K2** | G | N | - | F | I |
| **NI** | K | L | J | I | D |

Die Formulierungen wurden auf das Haar aufgetragen und von jeweils 5 Personen unabhängig voneinander bezüglich der untenstehenden Kriterien beurteilt. Dabei reicht die Beurteilungsskala von Note 1 (sehr gut) bis Note 5 (unbrauchbar), wobei die angegebenen Werte aus Mittelwertbildungen stammen. Die Auswertung der Resultate zeigt klar, daß Formylierung A (Zweierkombination aus amphoterem und saurem Polymer) die vorteilhafteste Rezeptur darstellt.

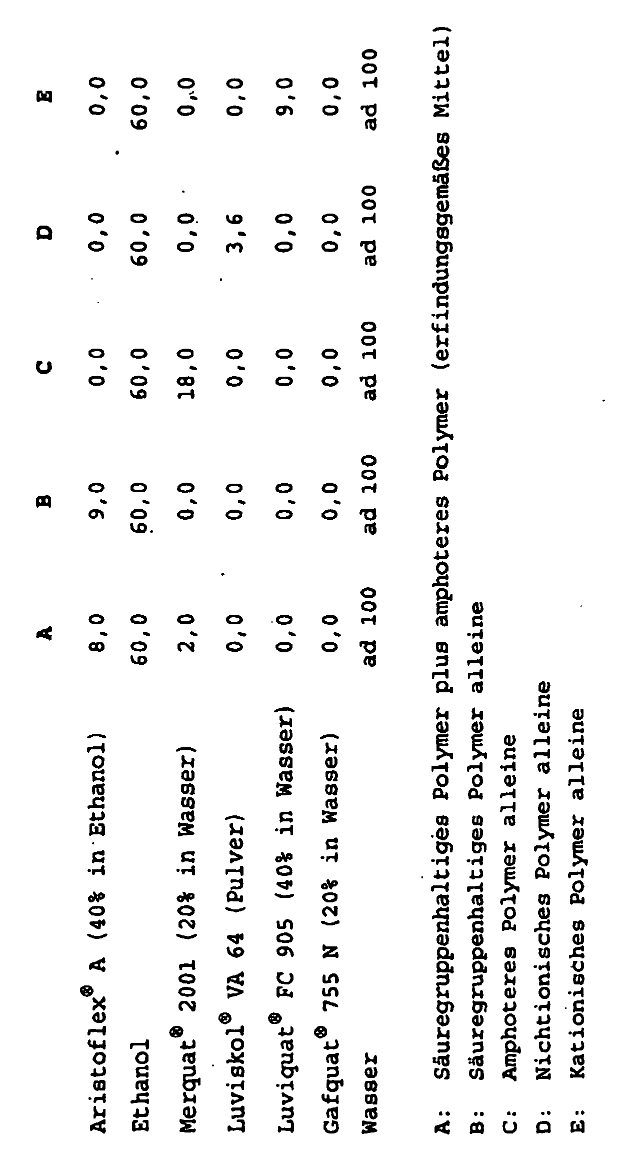

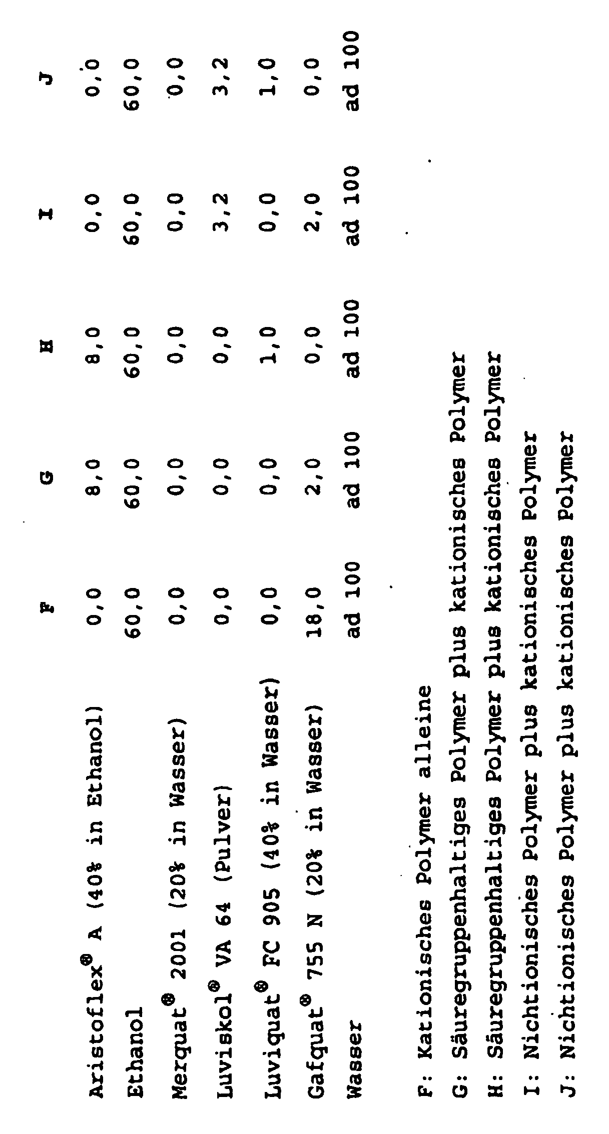

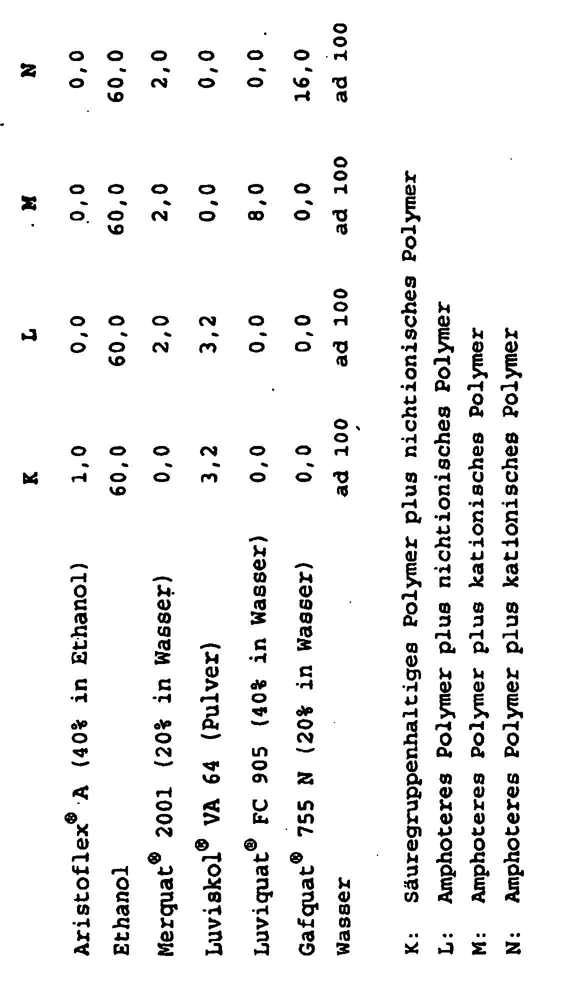

### Beurteilung der Rezepturen A bis N

| | **A** | **B** | **C** | **D** | **E** | **F** | **G** | **J** | **I** | **J** | **K** | **L** | **M** | **N** |
|---|---|---|---|---|---|---|---|---|---|---|---|---|---|---|
| Griff naß (1) | 1.3 | 4.1 | 5.0 | 3.8 | 3.1 | 5.0 | 1.8 | 2.3 | 2.5 | 2.3 | 3.7 | 1.8 | 3.4 | 5.0 |
| Kämmbarkeit | 1.5 | 4.4 | 1.7 | 4.5 | 1.2 | 2.9 | 1.7 | 2.2 | 2.5 | 2.3 | 4.3 | 2.2 | 2.1 | 3.2 |
| Klebrigkeit | 1.1 | 1.2 | 5.0 | 2.1 | 2.2 | 5.0 | 1.3 | 1.2 | 2.7 | 2.5 | 2.0 | 1.8 | 3.6 | 5.0 |
| Langzeithalt der Frisur | 1.9 | 2.2 | a | 2.5 | 3.8 | a | 1.9 | 2.7 | 2.2 | 2.3 | 2.3 | 2.0 | 4.2 | a |
| Elastizität/Volumen | 2.3 | 2.5 | a | 3.0 | 4.1 | a | 2.5 | 3.1 | 2.6 | 2.8 | 2.8 | 2.9 | 4.0 | a |
| Rauheit des Haares | 1.3 | 1.9 | a | 2.9 | 1.7 | a | 1.5 | 2.5 | 3.1 | 2.6 | 2.6 | 2.2 | 1.8 | a |
| Belastung/Rückstände | 1.0 | 1.0 | 5.0 | 3.3 | 2.1 | 5.0 | 1.2 | 1.2 | 1.7 | 1.9 | 2.9 | 2.4 | 2.9 | 5.0 |
| schlechtester Wert | 2.3 | 4.4 | 5.0 | 4.5 | 4.1 | 5.0 | 2.5 | 3.1 | 3.1 | 2.6 | 4.3 | 2.9 | 3.6 | 5.0 |
| Durchschnittswert | 1.5 | 2.5 | a | 3.1 | 2.6 | a | 1.7 | 2.2 | 2.5 | 2.4 | 2.9 | 2.2 | 3.1 | a |
| Mittlere Abweichung vom Durchschnitt | 0.5 | 1.3 | a | 0.8 | 1.1 | a | 0.4 | 0.7 | 0.4 | 0.3 | 0.8 | 0.4 | 0.9 | a |

| | | | | | | | | | | | | | | |
|---|---|---|---|---|---|---|---|---|---|---|---|---|---|---|
| (1) positiv: leicht gleitend; negativ: schmierig oder stumpf a nicht anwendbar, da zu starke Belastung des Haares | | | | | | | | | | | | | | |

## Patentansprüche

1. Haarfestigendes Mittel in Form eines Aerosol- oder Nonaerosol-Haarsprays, eines Haarschaumes, eines Haargeles, eines Haarwachses, einer Haarcreme oder einer Haarlotion mit einem Gehalt an
(A) mindestens einem amphoteren Polymer, welches ausgewählt ist aus Copolymeren von Acrylsäure, Methylacrylat und Methacrylamidopropyltrimethylammoniumchlorid, Copolymeren aus Acrylamidopropyltrimethylammoniumchlorid und Acrylaten, Copolymeren aus Acrylamid, Acrylamidopropyltrimethylammoniumchlorid, 2-Amidopropylacrylamidsulfonat und Dimethylaminopropylamin,
(B) mindestens einem Polymeren, welches keine quaternären Amingruppen enthält und gebildet ist aus mindestens einer Monomerart, welche Säuregruppen aufweist und wobei das Polymer ausgewählt ist aus vernetzten oder nicht vernetzten Vinylacetat/ Crotonsäure Copolymeren, Acrylsäure/Alkylacrylat/ N-Alkylacrylamid Copolymeren, partialveresterte Copolymere zwischen Vinylmethylether und Maleinsäureanhydrid und Vinylacetat/Crotonat/Vinylalkanoat Copolymeren,
wobei die Säuregruppen der Polymere (A) und (B) in neutralisierter oder in nicht neutralisierter Form vorliegen können;
ausgenommen einer Mousse-Zusammensetzung mit einem Gehalt an 6 Gew.% Ethanol, 4 Gew.% Butylester von Vinylmethylether/Maleinsäureanhydrid Copolymer (50%ig), 2 Gew.% PVP/VA (50%ig), 0,1 Gew.% Parfüm, 83,19 Gew.% Wasser, 0,1 Gew.% Aminomethylpropanol, 0,3 Gew.% Polyquaternium-47, 0,3 Gew.% Laurinsäureethanolamid, 0,1 Gew.% Steareth-21, 3 Gew.% Silikonmikroemulsion (25%ig) und 0,91 Gew.% Konservierungsmittel.

2. Mittel nach Anspruch 1, **dadurch gekennzeichnet, dass** es in Form eines Aerosol- oder Nonaerosol-Haarsprays, eines Haarschaumes, eines Haarwachses oder einer Haarlotion vorliegt und als amphoteres Polymer (A) ein Copolymer enthält aus Acrylsäure, Methacrylat und Methacrylamidopropyltrimethylammoniumchlorid.

3. Mittel nach einem der vorhergehenden Ansprüche, **dadurch gekennzeichnet, daß** es 0,01 bis 5 Gew.% des amphoteren Polymeren (A) enthält.

4. Mittel nach nach einem der vorhergehenden Ansprüche, **dadurch gekennzeichnet, daß** es 0,01 bis 20 Gew.% des Polymeren (B) enthält.

5. Mittel nach einem der vorstehenden Ansprüche, **dadurch gekennzeichnet, daß** die sauren Gruppen der Polymere (A) und (B) mit mindestens einem basischen Neutralisationsmittel neutralisiert sind.

6. Mittel nach einem der vorstehenden Ansprüche, **dadurch gekennzeichnet, daß** die sauren Gruppen der Polymere (A) und (B) zu 50 bis 100 Prozent neutralisiert sind.

7. Mittel nach einem der vorstehenden Ansprüche, **dadurch gekennzeichnet, daß** es auf wäßriger oder wäßrig-alkoholischer Basis konfektioniert ist.

8. Mittel nach einem der vorstehenden Ansprüche, **dadurch gekennzeichnet, daß** es zusätzlich mindestens eine kationische, kämmbarkeitsverbessernde Substanz enthält.

9. Mittel nach einem der vorstehenden Ansprüche, **dadurch gekennzeichnet, daß** es zusätzlich mindestens ein weiteres Polymer oder Copolymer enthält, ausgewählt aus der Gruppe der filmbildenden und haarfestigenden Polymere.

## Claims

1. Hair-setting agent in the form of an aerosol or nonaerosol hairspray, a hair foam, a hair gel, a hair wax, a hair cream or a hair lotion with a content of
(A) at least one amphoteric polymer which is chosen from copolymers of acrylic acid, methyl acrylate and methacrylamidopropyltrimethylammonium chloride, copolymers of acrylamidopropyltrimethylammonium chloride and acrylates, copolymers of acrylamide, acrylamidopropyltrimethylammonium chloride, 2-amidopropylacrylamide sulphonate and dimethylaminopropylamine,
(B) at least one polymer which contains no quaternary amine groups and is formed from at least one type of monomer which has acid groups, and where the polymer is chosen from crosslinked or uncrosslinked vinyl acetate/ crotonic acid copolymers, acrylic acid/alkyl acrylate/N-alkylacrylamide copolymers, partially esterified copolymers between vinyl methyl ether and maleic anhydride and vinyl acetate/crotonate/vinyl alkanoate copolymers,
where the acid groups of the polymers (A) and (B) may be present in neutralized form or in unneutralized form;
with the exception of a mousse composition with a content of 6% by weight of ethanol, 4% by weight of butyl ester of vinyl methyl ether/maleic anhydride copolymer (50% strength), 2% by weight of PVP/VA (50% strength), 0.1% by weight of perfume, 83.19% by weight of water, 0.1% by weight of aminomethylpropanol, 0.3% by weight of polyquaternium-47, 0.3% by weight of lauric acid ethanolamide, 0.1% by weight of steareth-21, 3% by weight of silicone microemulsion (25% strength) and 0.91% by weight of preservative.

2. Agent according to Claim 1, **characterized in that** it is present in the form of an aerosol or nonaerosol hairspray, a hair foam, a hair wax or a hair lotion and comprises, as amphoteric polymer (A), a copolymer of acrylic acid, methacrylate and methacrylamidopropyltrimethylammonium chloride.

3. Agent according to one of the preceding claims, **characterized in that** it comprises 0.01 to 5% by weight of the amphoteric polymer (A).

4. Agent according to one of the preceding claims, **characterized in that** it comprises 0.01 to 20% by weight of the polymer (B).

5. Agent according to one of the preceding claims, **characterized in that** the acidic groups of the polymers (A) and (B) are neutralized with at least one basic neutralizing agent.

6. Agent according to one of the preceding claims, **characterized in that** the acidic groups of the polymers (A) and (B) are 50 to 100 percent neutralized.

7. Agent according to one of the preceding claims, **characterized in that** it is formulated on an aqueous or aqueous-alcoholic basis.

8. Agent according to one of the preceding claims, **characterized in that** it additionally comprises at least one cationic, combability-improving substance.

9. Agent according to one of the preceding claims, **characterized in that** it additionally comprises at least one further polymer or copolymer chosen from the group of film-forming and hair-setting polymers.

## Revendications

1. Composition fixant les cheveux, sous forme d'une laque pour cheveux en aérosol ou non en aérosol, d'une mousse capillaire, d'un gel capillaire, d'une cire capillaire, d'une crème capillaire ou d'une lotion capillaire, ayant une teneur en :
(A) au moins un polymère amphotère qui est choisi parmi des copolymères d'acide acrylique, acrylate de méthyle et chlorure de méthacrylamidopropyltriméthylammonium, des copolymères de chlorure d'acrylamidopropyltriméthylammonium et acrylates, des copolymères d'acrylamide, chlorure d'acrylamidopropyltriméthylammonium, sulfonate de 2-amidopropylacrylamide et diméthylaminopropylamine,
(B) au moins un polymère qui ne contient pas de groupes amino quaternaires et est constitué d'au moins un type de monomère qui comporte des groupes acides, et le polymère étant choisi parmi des copolymères acétate de vinyle/acide crotonique, des copolymères acide acrylique/acrylate d'alkyle/N-alkylacrylamide, des copolymères partiellement estérifiés entre l'éther vinylméthylique et l'anhydride maléique et des copolymères acétate de vinyle/crotonate/alcanoate de vinyle, réticulés ou non réticulés,
les groupes acides des polymères (A) et (B) pouvant se trouver sous forme neutralisée ou sous forme non neutralisée ;
à l'exception d'une composition de mousse présentant une teneur de 6 % en poids d'éthanol, de 4 % en poids d'ester butylique de copolymère d'éther vinylméthylique/anhydride maléique (à 50 %), de 2 % en poids de PVP/VA (à 50 %), de 0,1 % en poids de parfum, de 83,19 % en poids d'eau, de 0,1 % en poids d'aminométhylpropanol, de 0,3 % en poids de polyquaternium-47, de 0,3 % en poids d'éthanolamide d'acide laurique, de 0,1 % en poids de stéareth-21, de 3 % en poids d'une microémulsion de silicone (à 25 %) et de 0,91 % en poids de conservateur.

2. Composition selon la revendication 1, **caractérisée en ce qu'**elle est présente sous la forme d'une laque pour cheveux en aérosol ou non en aérosol, d'une mousse capillaire, d'une cire capillaire ou d'une lotion capillaire, et comprend, en tant que polymère amphotère (A), un copolymère de l'acide acrylique, de méthacrylate et de chlorure de méthacrylamidopropyltriméthylammonium.

3. Composition selon l'une quelconque des revendications précédentes, **caractérisée en ce qu'**elle contient de 0,01 à 5 % en poids du polymère amphotère (A).

4. Composition selon l'une quelconque des revendications précédentes, **caractérisée en ce qu'**elle contient de 0,01 à 20 % en poids du polymère (B).

5. Composition selon l'une quelconque des revendications précédentes, **caractérisée en ce que** les groupes acides des polymères (A) et (B) sont neutralisés par au moins un agent de neutralisation basique.

6. Composition selon l'une quelconque des revendications précédentes, **caractérisée en ce que** les groupes acides des polymères (A) et (B) sont neutralisés à raison de 50 à 100 %.

7. Composition selon l'une quelconque des revendications précédentes, **caractérisée en ce qu'**elle est préparée à base aqueuse ou aqueuse-alcoolique.

8. Composition selon l'une quelconque des revendications précédentes, **caractérisée en ce qu'**additionnellement elle contient au moins une substance cationique améliorant l'aptitude au passage du peigne.

9. Composition selon l'une quelconque des revendications précédentes, **caractérisée en ce qu'**additionnellement elle contient au moins un autre polymère ou copolymère choisi dans le groupe des polymères filmogènes et fixant les cheveux.
